# EUROPEAN PATENT APPLICATION

(11) **EP 3 001 991 A1**
(43) Date of publication of application: **06.04.2016**
(21) Application number: 15187506.9
(22) Date of filing: 30.09.2015
(51) Int. Cl.: A61F 13/06

(54) **HEALTH CARE SOCK**

(30) Priority: 30.09.2014 MY PI2014702871
(71) Applicant: Salah, Mohammed Kamel, 21514 Jeddah (SA)
(72) Inventor: SALAH, Mohammed Kamel, 21514 Jeddah (SA)
(74) Representative: Schiweck, Weinzierl & Koch

(57) **Abstract**

The present invention discloses a health care sock having an interior cavity for receiving a human foot through an opening portion, comprising an outer layer (104) conforming to the shape of the foot (102), a toe portion (106), a heel portion (108) and a base (110) adjacent to the outer layer and conforms to the sole (130) of the foot (102) in order for the sole (130) to rest onto the base (110).

## Description

The present invention relates to daily necessities. More particularly, the present invention relates to a foot care product. Most particular, the present invention relates to a health care sock, which helps protects the foot of a user, improves the comfort level of a user and having anti-slip function for protecting the foot.

### BACKGROUND OF THE INVENTION

Starting in the 1980's and continuing to the present, significant advances in fiber technology have been applied to the sock industry, resulting in a wider variety of socks made for specialized use. Whatever the situation-whether it is running, camping, relaxing at home, or waiting out a long business meeting, donning a sock made with consideration of the movement and properties of the foot makes an astounding difference to overall comfort. Below is a general list of sock materials and their respective qualities.

Antimicrobial socks are treated with an agent that forms a molecular bond to the fibers themselves, acting as a barrier between the socks and the microorganisms attempting to land on them. These non-leaching antimicrobial agents reduce foot odor by impeding mold and mildew, and prolong the life of the sock by preventing excess bacteria from compromising the integrity of the fabric.

Acrylic fibers and yarns are generally characterized by their durability and their hydrophobic quality: unlike cotton socks, acrylic socks wick moisture away from the foot and reduces the risk of blisters. For this reason, acrylic socks are highly recommended for any athletic enterprise where shear force on the foot causes rubbing and possible irritation. Because acrylic socks do not absorb perspiration, they are naturally sterile and easy to clean without bleach. While some acrylics are more exclusive than others, all of the acrylic socks on TopSocks are woven with special attention to yarn quality and durability.

Bamboo's natural flexibility, softness, and lightness gave way to an ecologically-friendly fiber with astounding properties for the sock industry. The natural anti-bacterial and antiperspirant elements that allow Bamboo to proliferate in hot Asiatic climates translate into highly-durable socks that keep feet dry and hygienic. Recommended year-round for a variety of activities, these are exceedingly soft, comfortable socks, cool in the summer, and, as they are 100% biodegradable, they are as helpful to the environment as they are to the foot.

Cashmere speaks to the ultimate luxury in wool socks. These are indulgently soft, and can be appropriate for any climate, although they are especially useful in the winter due to their high moisture content and capacity for insulation.

Coolmax® is the quintessential fiber for athletic socks due to its superior wicking ability. The revolutionary brand from Invista is soft to the touch, and constructed with four channel cross-sectioning that moves perspiration away from the body to the outer surface of the fabric, where it evaporates before dampening the clothes. CoolMax® garments are breathable, lightweight, and preferred by top athletes around the world.

Cotton has been a material used in most casual and formal socks in recent history. Cotton socks are comfortable, have good stretch, and absorb moisture from the foot. Many high-quality socks are made with treated mercerized cotton to encourage an even softer texture and richer colors during the dyeing process.

Holofiber®, a revolutionary innovation which has proved through clinical testing to improve circulation and oxygenation by 10 to 18%, works by converting certain wavelengths of visible and invisible light into energy. This altered light energy, transmitted through the sock and into the skin, serves to oxygenate the body, and can improve the comfort and performance of athletes, speed the healing of ulcers, and ease pain from tired feet. Because Holofiber® is a responsive textile, some people have noted a warming or tingling sensation when wearing Holofiber® socks. This sensation indicates blood flow engaging the foot, and subsides when the body returns to a natural state of equilibrium. Socks with Holofiber® are especially useful for diabetics and those with ailments affecting the circulatory system.

Lycra® is the most popular form of stretch fiber, or spandex. An extremely versatile fabric, it dries quickly, retains its shape, and remains soft as it compresses the skin without restricting blood flow. Lycra® is an essential material that enables many socks to fit snugly and comfortably to the foot, and when strategically placed, can help relieve venous insufficiency and blood clotting in the feet and legs.

Merino Wool, the most highly-regarded wool used for socks, has the capacity to simultaneously wick moisture away from the foot while its inherent 8% moisture content moisturizes dry or cracked skin. Because Merino Wool is hypoallergenic, breathable, resilient, and wicks moisture even when damp, it is especially beneficial in socks worn in cold weather and outdoor winter activities.

Like other silk products, silk socks are lightweight with a smooth, soft texture that feels remarkable on the skin. Although silk is an incredibly strong natural fiber, silk socks are thin-often uses as sock liners-and they lose considerable strength when wet. Silk is utilized most often in formal footwear.

Thermax is a wicking polyester with a hollow core that traps air around the body and provides extra insulation, even when wet. Thermax is used in premium outdoor sport and ski performance socks.

Thanks to state-of -the-art fiber technology and construction techniques, modern socks have evolved into high-tech sports equipment with possible medical applications. Blisters, foot swelling, toenail damage, and pediatric bacteria have been the most pervasive afflictions compromising athletes' performance. All of these ailments are caused by the toll of shear force absorbed into skin when running, jumping, and pivoting, and all are exacerbated by cotton socks.

Because cotton retains rather than repels moisture, when cotton socks absorb sweat during exercise they swell, bunch, wrinkle, become less-breathable, and cause irregular friction that leads to blisters and foot bacteria. However, socks made from hydrophobic fibers, and which are constructed in a cross-sectional geometry, wick moisture away from the foot and encourage evaporation. In multiple studies cotton has been proven to absorb three times more moisture than acrylic, and fourteen times more moisture than CoolMax®. Once wet, cotton swells up to 45% and wool swells up to 35%, while acrylic fibers swell less than 5%. In ranking order, fibers that wick moisture away from the foot best are:
1 - CoolMax®
2 - Acrylic
3 - Polypropylene
4 - Wool
5 - Cotton

In view of the above, it is advantageous to provide a health sock comprising a combination of cotton, nylon and silicon, which helps protects the foot of a user, improves the comfort level of a user and having anti-slip function for protecting the foot.

### SUMMARY OF THE INVENTION

In order to overcome at least one of the above drawbacks of the prior art, the present invention aims to provide a health care sock.

To achieve the above purpose, the present invention provides a health care sock having an interior cavity for receiving a human foot through an opening portion, comprising:
an outer layer conforming to the shape of the human foot;
a toe portion;
a heel portion; and
a base adjacent to the outer layer and conforms to the sole of the foot in order for the sole to rest onto the base.

In an embodiment, the circumference outlining the opening portion comprises a first elastic member in order to facilitate the opening portion to receive the foot.

In another embodiment, the interior cavity of the sock receives the human foot therein, which leaves a portion of the leg above the heal portion to be exposed. Preferably, a second elastic member is attached to a portion at the circumference of the opening containing the first elastic member in order to surround the leg, hence holding the sock from slipping outside the foot. In this worn position, the space between the heel portion and the second elastic member is not covered by the sock

In also another embodiment, the toe portion includes a big toe portion and a general toe portion in order to separate the big toe and remaining toes of the human foot when the sock is worn.

In yet another embodiment, the base comprises an inner base layer, a middle base layer and an outer base layer. The middle base layer includes a gel layer positioned adjacent to the inner base layer and extends around an interior of such inner base layer. Preferably, the gel layer is a silicon gel layer.

In another embodiment, the bottom portion of the outer base layer is affixed with deformable members. Preferably, the deformable members are positioned directly on the bottom portion of the outer base layer, conforming to the length of the sole. With this configuration, the deformable members are in direct contact with the interior surface of the sole of the foot.

Preferably, the deformable members are a plurality of silicone pads affixed to the bottom portion of the outer base layer, which is an area corresponding to the sole of the human foot. On the other hand, the deformable members are in direct contact with the interior surface of the sole of a shoe when the sock is in use. The silicone pads are suitably deformable to provide a more gradual conforming of the sock to the contour of the human foot.

It is an advantage of the present invention to provide a health care sock that is comfortable in wearing and having non-slip property. The pair of socks has the characteristics of comfortability in wearing and wear resistance, good sweat absorption effect, anti-slip effect and capability of improving skin and heal cracked feet effect.

### BRIEF DESCRIPTION OF THE DRAWINGS

Figure 1 is a side elevational view illustrating the sock as applied to a human foot, according to a first embodiment of the present invention;
Figure 2 is a side elevational view illustrating the sock as applied to a human foot, according to a second embodiment of the present invention;
Figure 3 is an enlarged view of the toe portion of the sock, according to the present invention;
Figure 4 is a diagram of the sock showing its base layers, as applied to a human foot;
Figure 5 is a diagram illustrating the base layers of the sock, according to the present invention; and
Figure 6 is a diagram illustrating an outer layer of one of the base layers of the sock, according to the present invention.

### DETAILED DESCRIPTION OF THE INVENTION

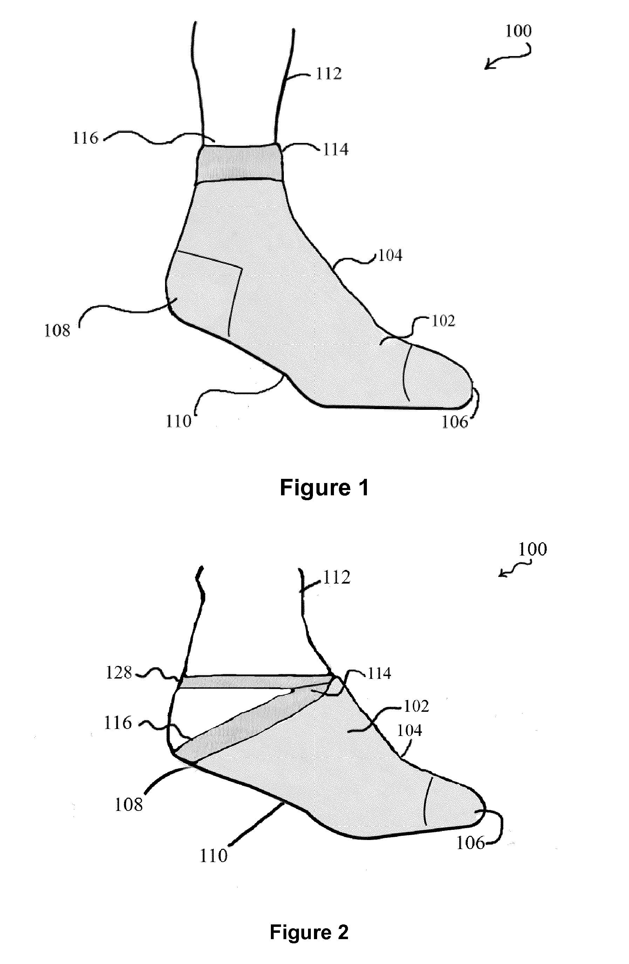

Referring to Figure 1, the health care sock **100** of the present invention is applied to a human foot **102.** The sock **100** according to this preferred embodiment is illustrated as having an outer layer **104** generally conforming to the shape of the human foot **102.** The sock **100** comprises a toe portion **106,** a heel portion **108** and a base **110.** The sock **100** of the present invention has an interior cavity suitable for receiving the human foot **102** therein. Leg **112** will extend upwardly and outwardly of the sock **100.** The sock **100** will be attached to the human foot in the manner of a conventional sock. The top of the body of the sock **100** is provided with an elastic rubber band **114** in order to facilitate the opening portion **116** when worn and the top of the socks after wearing tightening, so easy to slip the socks and enhances the wearing comfort. In an embodiment, the elastic rubber band **114** is contained in the circumference outlining the opening portion **116** in order to facilitate the opening portion **116** to receive the foot **102** and enhances wearing comfort.

Figure 2 shows a second embodiment of the sock **100** of the present invention, when applied to a human foot **102.** The sock according to this embodiment can be specially designed and advantageously used by Muslim men during Hajj & Omrah sessions in accordance with the rules and regulations of Muslim men's cloths during performing those two activities within a certain period of time.

In this embodiment, the interior cavity of the sock **100** receives the human foot **102** therein, which leaves the foot above the heal portion **108** to be exposed. An additional rubber band **128** is attached to a portion at the circumference of the opening **116** containing the rubber band **114** in order to surround the leg **112,** hence holding the sock **100** from slipping outside the foot **102.** In this worn position, the space between the heel portion **108** and the rubber band **128** is not covered by the sock **100.**

The sock **100** will be attached to the human foot **102** in this manner of which is in accordance with Muslim rules and regulation, which states that the portion of the Muslim man's foot should not be covered during performing Hajj or Omrah, which is the back side of the foot above the heal.

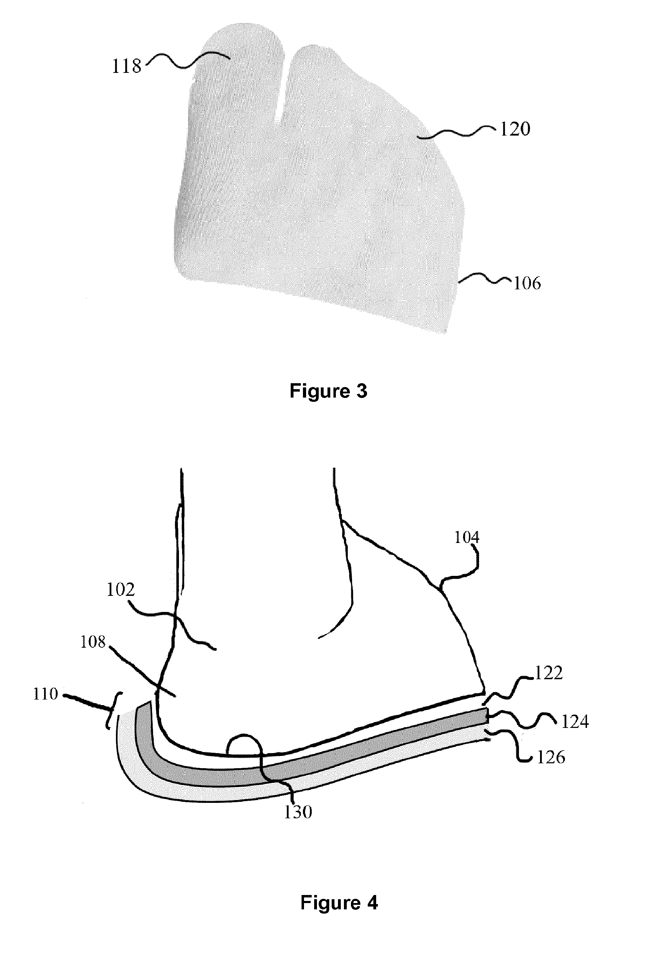

As shown in Figure 3, the toe portion **106** includes a big toe portion **118** and a general toe portion **120.** When the interior cavity receives the human foot **102,** the big toe portion **118** receives the big toe of the foot, while the general toe portion **120** receives the remaining toes of the human foot **102.** With this, the big toe and remaining toes of the human foot are separated when the sock is worn.

Referring to Figure 4, the outer layer **104** of the sock has a generally oval shape and conforms to the cross-sectional shape of the human foot **102.** The base **110** is positioned adjacent to the outer layer **104** and conforms to the sole **130** of the foot **102** in order for the sole **130** of the foot **102** to rest comfortably onto the base **110.** The base **110** comprises three layers namely inner base layer **122,** middle base layer **124** and outer base layer **126.**

Also referring to Figure 4, the inner base layer **122** is positioned adjacent to the sock outer layer **104** and extends at a predetermined length around an interior of the outer layer **104,** so as to conform to the sole **130** of the foot **102.** The middle base layer **124** is positioned adjacent to the inner base layer **122** and extends around an interior of such inner base layer **122.** Outer base layer **126** is positioned adjacent to the middle base layer **124** and extends around an interior of the middle base layer **124.**

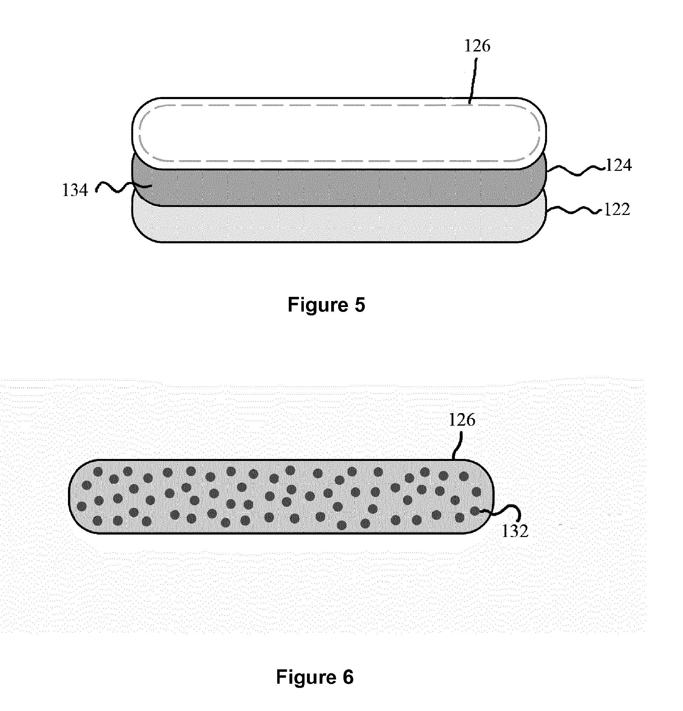

Figure 5 shows the base layer of the sock **100** comprising three (3) layers, namely inner base layer **122,** middle base layer **124** and outer base layer **126.** In a preferred embodiment, the middle base layer **124** includes a gel layer **134** positioned adjacent to the inner base layer **122** and extends around an interior of such inner base layer **122.** Preferably, the gel layer **134** is a silicon gel layer.

Referring to Figure 6, the bottom portion of the outer base layer **126** is affixed with deformable members **132.** The deformable members **132** are positioned directly on the bottom portion of the outer base layer **126,** conforming to the length of the sole **130.** With this configuration, the deformable members **132** are in direct contact with the interior surface of the sole of a shoe.

In a preferred embodiment, the deformable members **132** are a plurality of silicone pads affixed to the bottom portion of the outer base layer **126,** which is an area corresponding to the sole **130** of the human foot. On the other hand, the deformable members **132** are in direct contact with the interior surface of the sole of a shoe when the sock is in use, The silicone pads **132** are suitably deformable to provide a more gradual conforming of the sock to the contour of the human foot **102.** Hence, the present invention provides a sock **100** and shoe/sole **130** lining that suitable conforms to the contours of the human foot **102.** Within the concept of the present invention, various silicone pads **132** can be placed in the position as shown in Figure 6.

It is an advantage of the present invention to provide a sock **100** is capable of having anti-slip property. The anti-slip property is contributed by the gel layer **134** together with the inner base layer **122,** which sticks with the foot to give the ability of anti-slip which is part of the property of the gel.

It is another advantage of the present invention to provide a sock **100** that is comfortable to be worn. The comfort for the foot is contributed by the gel layer **134,** which acts as a soft carpet base, hence providing comfort during walking and long standing, as compared to a conventional sock.

It is also another advantage of the present invention to provide a sock having good sweat absorption effect. The sweat absorption property is contributed by the preferred sock fabric composition of the present invention comprising cotton 76.7%, polyester 21.3%, and elastane 2.0%, which result in moisture regain of 8.5% for cotton and 1.5% for both polyester and elastane. This is in accordance with European regulation EU 1007/2011, that provides comfortable sweat absorption effect to the foot.

It is a further advantage of the present invention to provide a sock **100** that is capable of improving skin and heal cracked feet effect. This property is contributed by the combination of both gel layer **134,** together with the inner base layer **122,** and the sock fabrics **100,** which protect the foot from heal crack. The heal **108** and the skin of the bottom portion of the foot **102** is surrounded with the gel layer when worn, which gives the skin and the heal **108** a comfortable zone and protects it from crack, as well as percentage of the regain of moisture that softens the skin.

It is yet another advantage of the present invention to provide a sock **100** having a big toe portion **118** and a general toe portion **120** which allows separation of the big toe from the remaining small toes. This separation allows the sock to be rigid and will not tilt, giving the user a comfortable feeling, and also allows the user to wear either a normal shoe or an open sandal (used mainly in Gulf countries and some other countries)

It is another advantage of the present invention to provide a sock **100** that leaves a certain part of the leg to be exposed, i.e. between the heel portion **108** and the rubber band **128.** This allows the sock **100** to be suitably worn by men during Hajj and/or Omrah, which is also in compliance with the Muslim rules and regulation, which states that the portion of the Muslim man's foot should not be covered during performing Hajj or Omrah, which is the back side of the foot above the heal.

The foregoing description of the present invention is for the purpose of illustration and explanation. Various changes in the details of the construction can be made within the scope of the claims without departing from the scope of the present invention.

## Claims

1. A health care sock (100) having an interior cavity for receiving a human foot (102) through an opening (116), wherein the sock (100) comprising:
an outer layer (104) conforming to the shape of the human foot (102);
a toe portion (106);
a heel portion (108); and
a base (110) adjacent to the outer layer (104) and conforms to the sole (130) of the foot in order for the sole (130) to rest onto the base (110).

2. A health care sock (100) according to Claim 1, wherein the circumference outlining the opening (116) comprises a first elastic member (114) in order to facilitate the opening (116) to receive the foot (102).

3. A health care sock (100) according to Claim 1, wherein the interior cavity of the sock (100) receives the human foot (102) therein, which leaves a portion of the leg above the heal portion (108) to be exposed.

4. A health care sock (100) according to Claim 1, further comprising a second elastic member (128) attached at a position relative to the circumference of the opening (116) containing the elastic member (114), thus surrounding the leg (112) and holding the sock (100) from slipping outside the foot (102).

5. A health care sock (100) according to Claim 1, wherein the toe portion (106) includes a big toe portion (118) and a general toe portion (120) in order to separate the big toe and remaining toes of the human foot (102) when the sock (100) is worn.

6. A health care sock (100) according to Claim 1, wherein the base (110) comprises an inner base layer (122), a middle base layer (124) and an outer base layer (126).

7. A health care sock (100) according to Claim 6, wherein the middle base layer (124) includes a gel layer (134) positioned adjacent to the inner base layer (122) and extends around an interior of the inner base layer.

8. A health care sock (100) according to Claim 6, wherein the gel layer (134) is a silicon gel layer.

9. A health care sock (100) according to Claim 6, wherein the bottom portion of the outer base layer (126) is affixed with deformable members (132), the deformable members (132) are in direct contact with the interior surface of the sole (130).

10. A health care sock (100) according to Claim 9, wherein the deformable members (132) are a plurality of silicone pads that conforms the sock (100) to the contour of the foot (102).
